# EUROPEAN PATENT SPECIFICATION

(11) **EP 3 960 214 B1**
(45) Date of publication and mention of the grant of the patent: **20.11.2024**
(21) Application number: 21186526.6
(22) Date of filing: 19.07.2021
(51) Int. Cl.: A61L 9/20

(54) **LIGHTING DEVICE HAVING FUNTION OF INACTIVATING BACTERIA OR VIRUSES**
BELEUCHTUNGSVORRICHTUNG MIT FUNKTION ZUR INAKTIVIERUNG VON BAKTERIEN ODER VIREN
DISPOSITIF D'ÉCLAIRAGE DOTÉ D'UNE FONCTION D'INACTIVATION DE VIRUS OU DE BACTÉRIES

(30) Priority: 01.09.2020 JP 2020147042; 15.06.2021 JP 2021099279
(43) Date of publication of application: 02.03.2022
(73) Proprietor: Ushio Denki Kabushiki Kaisha, Tokyo-to 100-8150 (JP)
(72) Inventor: NAITO, Keisuke, Tokyo, 100-8150 (JP); YAGYU, Hideaki, Tokyo, 100-8150 (JP); IGARASHI, Tatsushi, Tokyo, 100-8150 (JP); OHASHI, Hiroyuki, Tokyo, 100-8150 (JP)
(74) Representative: Maiwald GmbH

(56) References cited:
- WO-A1-2018/220161
- JP-A- H02 181 144
- JP-B2- 6 725 558
- KR-A- 20170 101 589
- US-B2- 10 094 522

## Description

### TECHNICAL FIELD

The present invention relates to a lighting device for inactivating bacteria or viruses.

### BACKGROUND ART

Conventionally, a lighting fixture embedded with a germicidal lamp, which is a box-shaped lighting fixture incorporating a germicidal lamp emitting ultraviolet light with a wavelength of 254 nm and a fluorescent lamp emitting light for illumination, has been proposed (see Patent Document 1) for the purpose of using in places in which food is handled on a daily basis, such as cooking areas.

### CITATION LIST

### PATENT DOCUMENT

Patent Document 1: JP-UM-A-63-187221
Patent Document 2: JP-A-2004-275070

### NON-PATENT DOCUMENT

Non-Patent Document 1: Shinji Tazawa, Yellow Lamps for Insects Pest Control, Journal of Science and Technology in Lighting Vol. 85, No. 3, 2001

### SUMMARY OF INVENTION

### Technical Problem

The germicidal lamp described in Patent Document 1, which emits ultraviolet light with a wavelength of 254 nm, has been commonly used for the purpose of sterilization. However, the ultraviolet light emitted from this germicidal lamp exhibits an insect attracting effect (also known as "insect attracting property"). For this reason, lighting a germicidal lamp embedded in the lighting fixture such as the one described in Patent Document 1, especially at night, is likely to attract insects to the fixture in response to the ultraviolet light emitted from the germicidal lamp. Attracting insects is a subject that should be avoided in places where hygiene problems may arise, especially in places where food or the like are handled.

Furthermore, besides the places where food or the like are handled, there is a need to avoid attracting insects in outdoor areas where people may pass by or stop nearby, for aesthetic reasons as well as for hygienic reasons.

In view of the above issue, it is an object of the present invention that provides a lighting device for inactivating bacteria or viruses, the device being capable of inactivating bacteria or viruses while inhibiting the attraction of insects.

### Solution to problem

A lighting device for inactivating bacteria or viruses of the present invention, includes: a first light source for emitting ultraviolet light with a peak wavelength in a wavelength band of 200 nm or more and less than 240 nm and with light intensity suppressed in a wavelength band of 250 nm or more and less than 400 nm; and
a second light source that is composed of an LED element, for emitting white light for illumination.

Hereinafter, each term used in this specification is defined.
<1 > The "inactivation" refers to a concept that encompasses the killing of bacteria or viruses, or the loss of their infectivity or toxicity.
<2> The "bacteria" refers to microorganisms including fungi (mold).
<3> The "light intensity suppressed" means that the light intensity at the light-irradiating surface is 1 mW/cm² or less and is also less than 5% with respect to the light intensity at the peak wavelength, preferably less than 3%.
<4> The "white light" does not refer to monochromatic light such as blue light, green light, and red light, instead refers to light with a spectrum that includes wavelength components belonging to the blue light wavelength range (430 nm or more and less than 500 nm), green light wavelength range (500 nm or more and less than 600 nm), and red light wavelength range (600 nm or more and less than 800 nm). The boundaries between these wavelength components in the white light can be distinct or unclear.

Hereinafter, "bacteria or viruses" may be collectively referred to as "germs".

US 20170284609 A1 describes a light-emitting device comprising a layered structure containing a photoluminescent layer. The photoluminescent material is excited by excitation light of 450 nm and emits highly polarized light of from 500 to 700 nm and particularly about 600 nm. JP 6 725558 B2 discloses an ultraviolet sterilizer that switches between ultraviolet light and visible light. The sterilizer utilizes two LEDs, one emitting ultraviolet light at a peak wavelength of 260 nm to 290 nm and the other at a peak wavelength of about 380 nm. Low-pressure mercury lamps are used as ultraviolet lamps emitting ultraviolet light of 254 nm wavelength, which are generally used for sterilization applications, including the ultraviolet lamps described in Patent Document 1. FIG. 1 is a graph illustrating the emission spectrum of the low-pressure mercury lamp.

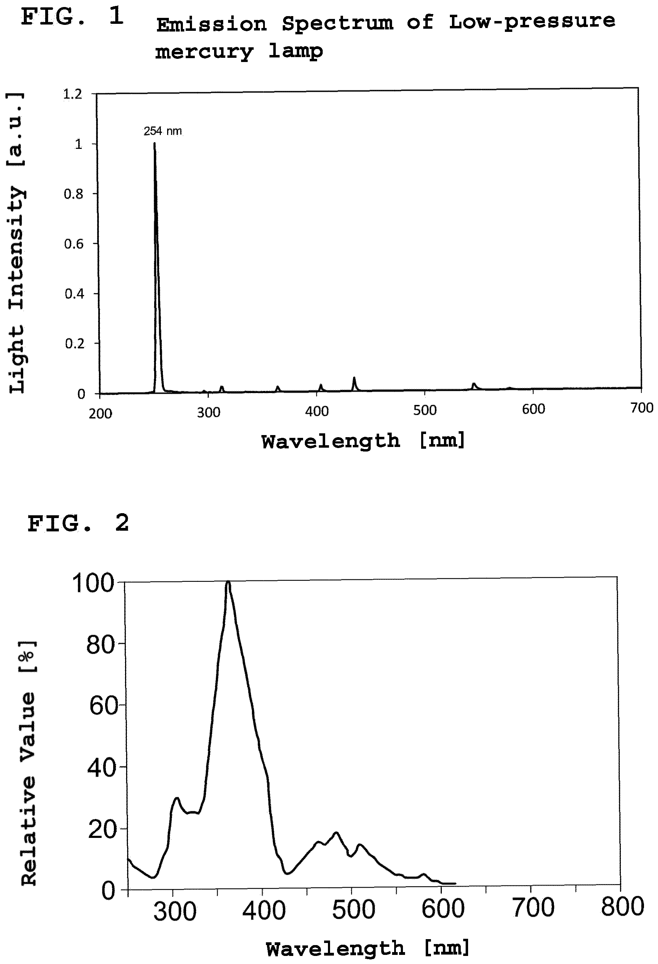

As shown in FIG. 1, the low-pressure mercury lamp strongly emits ultraviolet light with a wavelength of 254 nm, which is within the visual sensitivity range of insects, and also emits ultraviolet light in a wavelength band of 300 nm or more and less than 400 nm in addition to the ultraviolet light with a wavelength of 254 nm. Many insects have high visual sensitivity to ultraviolet light in this wavelength band of 300 nm or more and less than 400 nm. FIG. 2 is a graph illustrating the visual sensitivity of Drosophila as a representative of such insects (see Non-Patent Document 1). FIG. 2 confirms that Drosophila exhibits high visual sensitivity in a wavelength band of 300 nm or more and less than 400 nm.

The Patent Document 2 also states that many insects are strongly attracted to ultraviolet light with a wavelength of 340 nm to 380 nm.

As described above in the "Technical Problem", the lighting fixture embedded with a germicidal lamp described in Patent Document 1 attracts insects by the ultraviolet light belonging to a wavelength band of 250 nm or more and less than 400 nm, which is emitted from the germicidal lamp installed in the lighting fixture, and furthermore, strongly attract insects by the ultraviolet light belonging to a wavelength band of 300 nm or more and less than 400 nm.

In contrast, the lighting device for inactivating bacteria or viruses (hereinafter simply referred to as "lighting device with inactivation function") of the present invention is provided with a first light source emitting ultraviolet light for inactivation, the ultraviolet light having a peak wavelength in a wavelength band of 200 nm or more and less than 240 nm, and having light intensity in a wavelength band of 250 nm or more and less than 400 nm being suppressed. In other words, the ultraviolet light emitted from the first light source has light intensity suppressed in the wavelength band of 250 nm or more and less than 400 nm, the wavelength band that exhibits insect attracting effect (insect attracting property). Specifically, the light intensity of the first light source on the light-irradiating surface from which the ultraviolet light is emitted, is 1 mW/cm² or less, and the light intensity thereof is less than 5% with respect to the light intensity of a peak wavelength in a wavelength band of 200 nm or more and less than 240 nm. This configuration effectively reduces the insect attracting property.

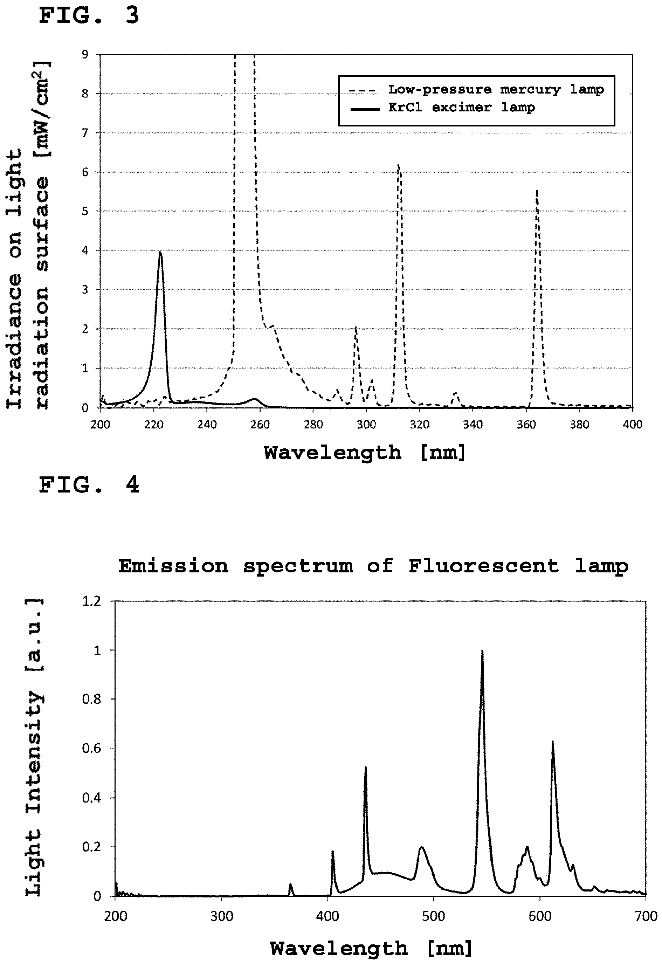

FIG. 3 is a graph illustrating the irradiation spectra of the KrCl excimer lamp, which is an example of the first light source, and the low-pressure mercury lamp shown in FIG. 1, in a superimposed manner, at the light-irradiating surface when the same power is supplied to both the lamps. FIG. 3 shows that ultraviolet light emitted from the low-pressure mercury lamp has a light intensity peak at a level far exceeding 1 mW/cm² in a wavelength band of 300 nm or more and less than 400 nm.

In contrast, in the case of KrCl excimer lamps, the light intensity in a wavelength band of 250 nm or more and less than 400 nm is less than 5% with respect to the light intensity at the peak wavelength, and is suppressed to less than 1 mW/cm². In FIG. 3, the light intensity at the peak wavelength of the KrCl excimer lamp is lower than that of the low-pressure mercury lamp; this intensity difference is explained by the characteristics that, at present, the low-pressure mercury lamp has a higher light conversion efficiency than the KrCl excimer lamp.

In FIG. 3, the KrCl excimer lamp is given as an example for convenience of explanation; however the first light source can be any light source having a peak wavelength in a wavelength band of 200 nm or more and less than 240 nm, and having a light intensity in a wavelength band of 250 nm or more and less than 400 nm that is less than 5% with respect to the light intensity at the peak wavelength, and less than 1 mW/cm², similar to this KrCl excimer lamp.

Ultraviolet light in a wavelength band of 240 nm or more and less than 300 nm is known to pose a risk to the human body when irradiated thereto. The skin is divided into three sections, in order of proximity from near its surface: epidermis, dermis, and its deeper subcutaneous tissue. The epidermis is further divided into four layers in order of proximity from near its surface: stratum corneum, stratum granulosum, stratum spinosum, and stratum basale. When the human body is irradiated with ultraviolet light in the wavelength band of 240 nm or more and less than 300 nm, such as 254 nm as a germicidal ray, the light penetrates the stratum corneum, reaches the stratum granulosum or the stratum spinosum, or in some cases the stratum basale, and is absorbed by the DNA of the cells in these layers, thus resulting in the risk of skin cancer.

In contrast, ultraviolet light in a wavelength band of 200 nm or more and less than 240 nm (more preferably, ultraviolet light in a wavelength band of 200 nm or more and less than 235 nm), when irradiated to the human body, is absorbed by the stratum corneum of the skin and does not penetrate further inward (the stratum basale side). Keratinocyte in the stratum corneum is a cell without a nucleus, thereby it does not have DNA as does, for example, a squamous cell. Hence, the light with this particular wavelength band poses a low risk of destroying DNA due to absorption of light by cells, unlike the case of ultraviolet light in a wavelength band of 240 nm or more and less than 300 nm being irradiated. Furthermore, the light intensity in the band of 235 nm or more and less than 240 nm is also suppressed, thereby ensuring that the risk of damaging DNA is reduced due to absorption of ultraviolet light by cells. Therefore, it is more preferable that the ultraviolet light for inactivation emitted from the first light source have a peak wavelength in a wavelength band of 200 nm or more and less than 235 nm, and have the light intensity in a wavelength band of 240 nm or more and less than 400 nm being suppressed.

As described above, the ultraviolet light emitted from the first light source has its light intensity suppressed in the wavelength band of 250 nm or more and less than 300 nm, in addition to the wavelength band of 300 nm or more and less than 400 nm. This configuration effectively reduces the insect attractive property and the adverse effect on the human body even if the first light source is turned on during the time when humans are present near the lighting device.

The ultraviolet light having the light intensity suppressed in the wavelength band of 240 nm or more and less than 400 nm further effectively reduces its adverse effect on the human body. Furthermore, the ultraviolet light having the light intensity suppressed in the band of 235 nm or more and less than 400 nm more reliably reduce its adverse effect on the human body while reducing its insect attracting property.

Irradiating ultraviolet light emitted from the first light source in the wavelength band of 200 nm or more and less than 240nm is capable of inactivating the germs in the irradiated area. More preferably, irradiating ultraviolet light having the wavelength band of 200 nm or more and less than 235 nm is capable of inactivating germs present in the irradiated area while reliably reducing the adverse effect on the human body. Suppressing the wavelength band is achieved, for example, by selecting an appropriate light source or by using an optical filter that suppresses the band concerned.

Incidentally, fluorescent lamps, which emit light for illumination, are known to contain light in the ultraviolet band in addition to visible light band. FIG. 4 is a graph illustrating the emission spectrum of a typical fluorescent lamp. In the example shown in FIG. 4, the light-emitting color of the fluorescent lamp is daylight white color, which is commonly and widely used as the fluorescent lamp.

The spectrum in FIG. 4 confirms that the light emitted from the fluorescent lamp also contains ultraviolet light in a wavelength band of 300 nm or more and less than 400 nm. Hence, the ultraviolet light contained in the light emitted from the fluorescent lamp may attract insects.

In contrast, the lighting device with inactivation function of the present invention is provided with a second light source that is composed of an LED element that emits white light for illumination. In principle, the LED element can reduce the intensity of ultraviolet light in a wavelength band of 300 nm or more and less than 400 nm compared with a fluorescent lamp.

In other words, according to the lighting device with inactivation function of the present invention, the first light source emits the ultraviolet light for inactivation in the wavelength band having low insect attracting property and serving to inactivate germs, and the second light source emits the white light for illumination from an LED element having low insect attracting property. This configuration enables a lighting device for inactivating germs, and significantly reducing insect attracting property compared to a conventional lighting fixture embedded with a germicidal lamp.

This type of lighting device for inactivating germs is expected to be used in places where humans are nearby and hygiene control is highly required, such as food factories and cafeterias, and also in places where the outside air is likely to be exposed and unspecified people may approach. Examples of the latter include train stations, outdoor plazas, outdoor stadiums, outdoor theme parks, vehicles (cabs, trains, and buses), facility entrances and reception areas, and vending machines.

The lighting device with inactivation function according to the present invention provides sterilization and virus inactivation capability that are inherent in ultraviolet light without causing erythema or keratitis in the skin or eyes of humans or animals. In particular, unlike conventional ultraviolet light sources, the lighting device with inactivation function has a feature of operating in a manned environment. This feature enables the lighting device with inactivation function, when installed in an indoor or outdoor manned environment, to irradiate the entire environment and provide inactivation and sterilization of viruses in the air and on the surface of the components equipped in the environment.

This feature corresponds to the Goal 3 of the United Nations-led Sustainable Development Goals (SDGs) "Ensure healthy lives and promote well-being for all at all ages", and also significantly contributes to achieving the Target 3.3 "By 2030, end the epidemics of AIDS, tuberculosis, malaria and neglected tropical diseases and combat hepatitis, water-borne diseases and other communicable diseases".

The first light source may irradiate an area with the ultraviolet light, the area being irradiated by the second light source.

In the space where humans work under the white light from the second light source, this configuration enables both the illumination of the space and the inactivation of germs in the space. As described above, the ultraviolet light from the first light source has a minimal adverse effect on the human body. Hence, even if humans using white light illumination is in the vicinity of the lighting device during the time when both the first and the second light sources activate, the lighting device inactivates germs, with inhibiting the adverse effect on the human body. In addition, with the effect of insect attraction being inhibited, the lighting device is likely to prevent the humans from feeling discomfort by seeing a large number of insects.

The second light source may irradiate an object operated by a person with the white light, whereas the first light source may irradiate the object with the ultraviolet light to inactivate bacteria or viruses attached to the object.

Examples of the above object include the operation buttons or the touch panels of ticket vending machines for admission tickets, boarding tickets, meal tickets, and vending machines or the like (hereinafter collectively referred to as "operation units"). For example, such objects that are intended to be operated by person's fingers are prone to adhesion of normal bacteria flora including Staphylococcus epidermidis or Micrococcus, which are present on person's fingers, or other bacteria. According to the above lighting device with inactivation function, the first light source irradiates the object with ultraviolet light for inactivation, thus inactivating bacteria attached to the object.

In addition, light for illumination is usually irradiated onto operation units including operation buttons and touch panels, since they need to be operated by a person. According to the above lighting device with inactivation function, the second light source irradiates the operation units including operation buttons and touch panels with white light for illumination, thus using the light as illumination for the operation.

As described above, the ultraviolet light for inactivation emitted from the first light source and the white light for illumination emitted from the second light source are both light that inhibits insect attracting property, thus inhibiting the side effect of attracting insects to the object during inactivation of germs or illumination.

The term "object operated by a person" includes a scale that functions when a person places his or her foot on a predetermined spot, or a visual acuity meter that functions when a person places his or her chin on a predetermined spot and looks into it. In other words, the above object may include a case in which a human body part other than fingers, clothes that cover the human body, belongings or the like touch the object.

The lighting device with the function of inactivating bacteria or viruses may be provided with a control unit for controlling to light the first light source and the second light source, and the control unit may control to repeatedly turn on and turn off the first light source within a lighting-off period of the second light source.

There may be a case in which the inactivation process of germs needs to be continued even when the second light source that emits white light for illumination is turned off. For example, even in an unmanned environment, there is a possibility that microorganisms such as mold and bacteria can proliferate, or that microorganisms and viruses from the outside world can invade if the environment is open to the outside air. For another example, there may be a case in which a ticket vending machine described above no longer needs to be irradiated in non-operating hours (i.e., non-business hours); however the ticket vending machine needs to continue the inactivation process of germs that may be attached thereon.

The illuminance of the area where the lighting device with the function of inactivating germs is installed, is relatively lower during the lighting-off period of the second light source, compared with that during a lighting-on period of the second light source. Hereinafter a case in which the first light source is turned on under a relatively low space illuminance (e.g., the space illuminance is less than 50 [lx]) is considered. In this case, the effect of attracting insects can occur even when the first light source emits ultraviolet light that inhibits insect attracting property, compared to the case in which the first light source is turned on under relatively high illumination in the space. The reason for this phenomenon is inferred as follows.

Many insects possess phototaxis, and tend to be attracted to light sources that are perceived to be relatively bright. For example, if the light source has even a small amount of emission intensity in the ultraviolet light in a wavelength band of 250 nm to 400 nm or in the visible light in a wavelength band of 400 nm to 550 nm, the insects will perceive the light from the light source as relatively brighter in a darker surrounding environment, thus even a faint light may be noticeable to the insects.

The ultraviolet light from the first light source is intended to have light intensity suppressed in the band of 250 nm or more and less than 400 nm, which exhibits the effect of attracting insects; however, the light intensity of the band is not completely zero and may have a faint light intensity. In the case of the first light source being a discharge lamp, the discharge phenomenon produces a faint visible light, which relatively stands out in dark surroundings, thus presumably attracting insects to the faint light.

As for the configuration described above, the first light source, in contrast, is controlled to be turned on and off repeatedly during the lighting-off period of the second light source. Hence, although the relative illuminance decrease in surroundings causes insects to be attracted to the first light source during its lighting, the insects may lose sight of the source (target) of the ultraviolet light when the first light source is turned off, thus unlikely to be attracted toward the lighting device.

The extent to which germs is inactivated depends on the amount of ultraviolet light dosage (accumulated irradiation amount) to the area to be inactivated. Hence, even if the first light source is controlled to be turned on and off repeatedly, the inactivation of germs is achieved as long as the lighting is repeated.

The control unit may control to repeatedly turn on and off the first light source, with a lighting-on period of the first light source being set to 60 seconds or less, and a lighting-off period of the first light source being set to a time longer than the lighting-on period thereof.

The above configuration further enhances the effect of causing insects to lose sight of the ultraviolet light source.

The control unit may control to repeatedly turn on and off the first light source with the lighting-on period of the first light source being set to 50% or less with respect to the lighting-off period of the first light source.

The above configuration further enhances the effect of causing insects to lose sight of the ultraviolet light source. It is more preferable that the lighting-on period of the first light source be set to 25% or less with respect to the lighting-off period of the first light source.

The lighting device with the function of inactivating bacteria or viruses may be provided with a control unit that controls to light the first light source and the second light source. The control unit may control the first light source to provide the lighting-off period of the first light source within a lighting-on period of the second light source.

The first light source described above is a light source that emits ultraviolet light for inactivation with a peak wavelength in the wavelength band of 200 nm or more and less than 240 nm. Hence, although this ultraviolet light is irradiated to bacteria possessing a photoreactivation function in an environment under which white light for illumination from a second light source is being irradiated (lighting environment), the photoreactivation function is inhibited. Thereby, the effect of inactivation maintains without continuously lighting the first light source. This issue will be discussed later in the "DESCRIPTION OF EMBODIMENTS".

In this case, the control unit may control repeatedly to turn on and off the first light source.

The first light source may include an excimer lamp that contains Kr and Cl as light-emitting gases. This enables the first light source to emit ultraviolet light that has a spectrum with a peak wavelength near 222 nm and a half value width of approximately 15 nm.

The first light source may include an excimer lamp that contains Kr and Br as light-emitting gases. This enables the first light source to emit ultraviolet light that has a spectrum with a peak wavelength near 207 nm and a half value width of approximately 15 nm.

In the case that the ultraviolet light generated by the first light source contains a certain amount of light intensity in a wavelength band of 250 nm or more and less than 400 nm, the first light source may be provided with a filter that blocks the propagation of light in the wavelength band thereof.

[Advantageous Effects of Invention] The lighting device with a function of inactivating bacteria or viruses enables both visible light illumination and inactivation of germs, while inhibiting more the effect of attracting insects than conventional systems.

### BRIEF DESCRIPTION OF DRAWINGS

FIG. 1 is a graph illustrating the emission spectrum of a low-pressure mercury lamp.
FIG. 2 is a graph illustrating the visual sensitivity of Drosophila.
FIG. 3 is a graph illustrating the irradiation spectra of a KrCl excimer lamp, which is an example of the first light source, and the low-pressure mercury lamp, at the light-irradiating surface when the same power is applied to both the lamps.
FIG. 4 is a graph illustrating the emission spectrum of a conventional fluorescent lamp.
FIG. 5 is a schematic view of a lighting device with a function of inactivating bacteria or viruses illustrating an embodiment of the present invention.
FIG. 6 is a perspective appearance view of a lighting device with a function of inactivating bacteria or viruses illustrating an embodiment.
FIG. 7 is a functional block diagram schematically illustrating an internal configuration of a lighting device with a function of inactivating bacteria or viruses.
FIG. 8 is a schematic diagram illustrating an example of the appearance of the first light source.
FIG. 9 is a schematic exploded view of the first light source shown in FIG. 8.
FIG. 10 is a plan view schematically illustrating the positional relation between the excimer lamp and electrode blocks in the first light source shown in FIG. 8.
FIG. 11 is a graph illustrating an example of the spectrum of ultraviolet light emitted from the first light source.
FIG. 12A is a graph illustrating a verification result explaining that the ultraviolet light emitted from the first light source is effective in inactivating bacteria.
FIG. 12B is a graph illustrating a verification result that explaining the ultraviolet light emitted from the first light source is effective in inactivating viruses.
FIG. 13 is a graph illustrating an example of the spectrum of white light emitted from the second light source.
FIG. 14 is a schematic view of a lighting device with a function of inactivating bacteria or viruses illustrating another embodiment of the present invention.
FIG. 15 is a graph illustrating the verification result explaining that the intermittent lighting of the first light source is effective in inactivating bacteria.
FIG. 16 is a graph illustrating the absorption spectra of FAD (flavin adenine dinucleotide) and riboflavin.
FIG. 17A is a graph illustrating the evaluation result explaining the extent of photoreactivation of bacteria under visible light with respect to ultraviolet light irradiated from a low-pressure mercury lamp.
FIG. 17B is a graph illustrating the evaluation result explaining the extent of photoreactivation of bacteria under visible light with respect to ultraviolet light irradiated from a KrCl excimer lamp.
FIG. 18A is a graph illustrating the evaluation result explaining the extent of photoreactivation of bacteria under visible light with respect to ultraviolet light irradiated from a KrBr excimer lamp.
FIG. 18B is a graph illustrating the emission spectrum of a KrBr excimer lamp.
FIG. 19 is a graph illustrating the characteristics of the average absorption coefficient of protein in the wavelength band of ultraviolet light.
FIG. 20 is a graph illustrating the absorption spectrum of Escherichia coli (E. coli).

### DESCRIPTION OF EMBODIMENTS

Embodiments of a lighting system a function of inactivating bacteria or viruses will be described with reference to the drawings as appropriate. It is noted that following each drawing is merely schematically illustrated; the dimensional ratios on the drawing do not necessarily match the actual dimensional ratios. Also the dimensional ratios between each drawing do not necessarily match either.

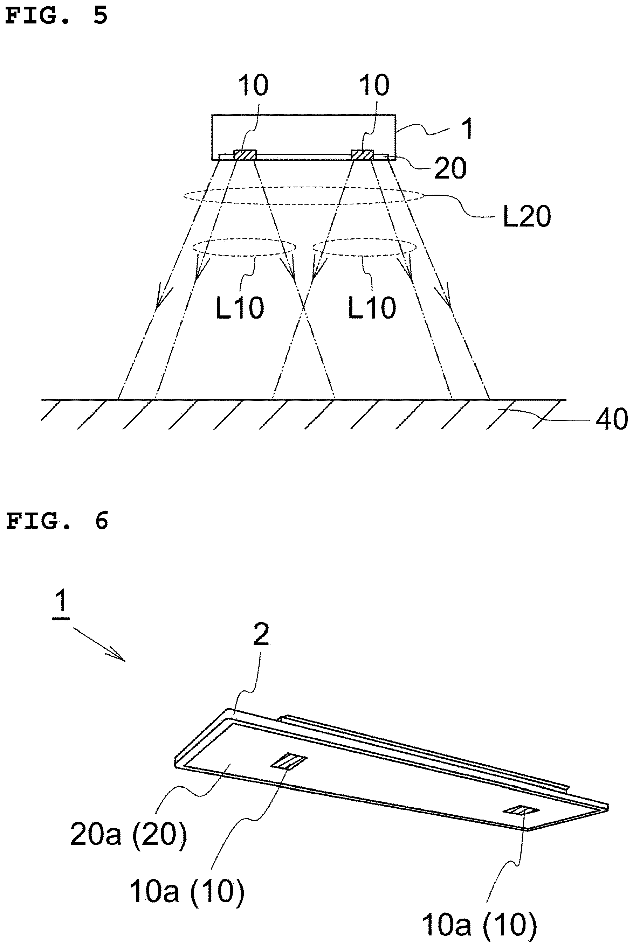

FIG. 5 is a schematic view of a lighting device with a function of inactivating bacteria or viruses illustrating an embodiment of the present invention. As shown in FIG. 5, the lighting device with inactivation function 1 is provided with a first light source 10 and a second light source 20. As described below, the first light source 10 emits ultraviolet light L10 for inactivation, and the second light source 20 emits white light L20 for illumination. In the example shown in Fig. 5, both of the ultraviolet light L10 and the white light L20 are schematically illustrated to be irradiated to a target area 40 from the lighting device with inactivation function 1.

FIG. 6 is a perspective appearance view of a lighting device with a function of inactivating bacteria or viruses illustrating an embodiment. In the embodiment shown in FIG. 6, the lighting device with inactivation function 1 is provided with a casing 2 that houses the first light source 10 and the second light source 20. The surface of the casing 2 includes a light extraction surface 10a of the ultraviolet light L10 from the first light source 10 and a light extraction surface 20a of the white light L20 from the second light source 20.

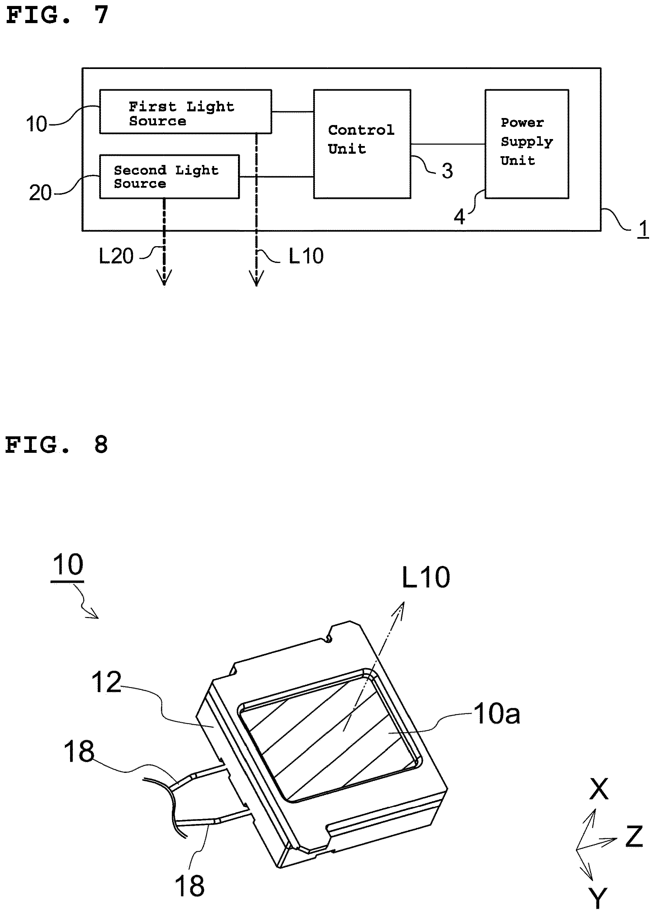

FIG. 7 is a functional block diagram schematically illustrating an internal configuration of the lighting device with the inactivation function 1. As shown in FIG. 7, the lighting system with the inactivation function 1 is provided with a control unit 3 that controls to light the first light source 10 and the second light source 20, and a power supply unit 4 that supplies power for lighting the first light source 10 and the second light source 20. The power supply unit 4 includes a power circuit for converting the voltage applied from a power source (not shown) into the voltage required for lighting. The control unit 3 controls whether or not to supply the voltage generated by the power supply unit 4 to the first light source 10 and the second light source 20.

In FIG. 7, the power supply unit 4 is illustrated to be connected to the control unit 3 through a single line; however, the line is just schematically illustrated. In practice, the power supply unit 4 generates each of the voltage signals required to light the first light source 10 and the second light source 20, and the voltage signal each can be independently supplied to the corresponding light sources (10, 20) through the control unit 3.

The control unit 3 performs the lighting control for the first light source 10 and the lighting control for the second light source 20 independently from each other. An example of the control contents performed by the control unit 3 will be described later.

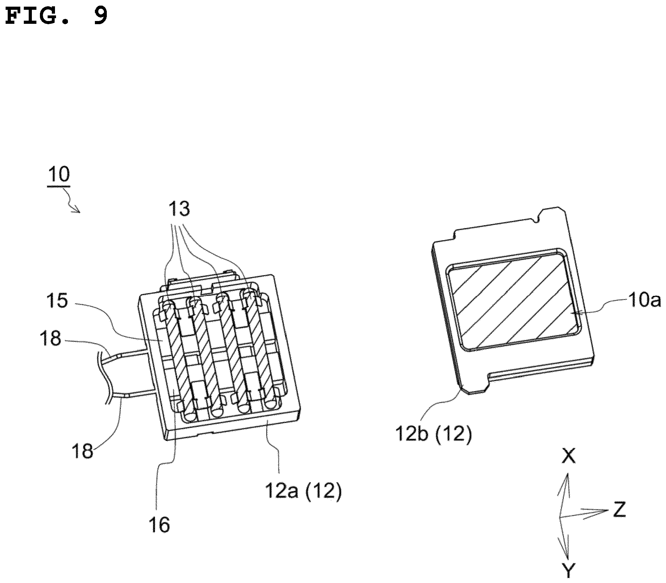

FIG. 8 is a schematic diagram illustrating an example of the appearance of the first light source 10. FIG. 9 is a schematic exploded view of the lamp house 12 of the first light source 10 shown in FIG. 8, disassembling a main body casing part 12a and a lid part 12b.

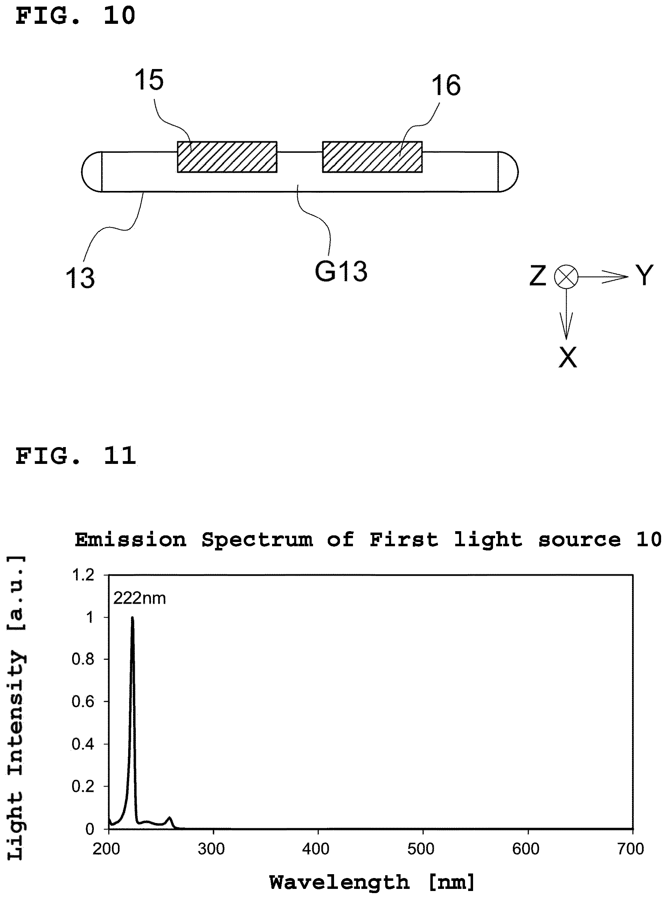

In the following FIGs. 8 to 10, the description is given with reference to the X-Y-Z coordinate system, in which the extraction direction of the ultraviolet light L10 is the X direction and the plane orthogonal to the X direction is the YZ plane. In more detail, the direction of the tube axis of the excimer lamp 13 is the Y direction, and the direction perpendicular to the X and Y directions is the Z direction, as described below with reference to FIGs. 9 and 10.

As shown in Figs. 8 and 9, the first light source 10 is provided with the lamp house 12 having the light extraction surface 10a on its one side. The lamp house 12 is provided with the main casing part 12a and the lid part 12b. The main casing part 12a houses excimer lamps 13 and electrode blocks (15, 16). In FIG. 9, a case in which the lamp house 12 houses the four excimer lamps 13 is illustrated as an example. The electrode blocks (15, 16) are electrically connected to power feed wires 18 and constitute electrodes for feeding power to each of the excimer lamps 13. The power feed wires 18 are connected to the power supply unit 4 (see FIG. 7).

FIG. 10 is a plan view schematically illustrating the positional relation between the excimer lamp 13 and the electrode blocks (15, 16).

As shown in FIGs. 8 to 10, the first light source 10 according to this embodiment is provided with the two electrode blocks (15, 16) that are arranged to be in contact with the outer surfaces of the light-emitting tubes of the respective excimer lamps 13. The electrode blocks (15, 16) are spaced apart in the Y direction. The electrode blocks (15, 16) are made of a conductive material, preferably a material that is reflective to the ultraviolet light L10 emitted from the excimer lamp 13. Examples of the material of the electrode blocks (15, 16) include Al, Al alloy, or stainless steel. In this embodiment, both of the electrode blocks (15, 16) are arranged to straddle each excimer lamp 13 with respect to the Z direction, while being in contact with the outer surface of the light-emitting tube of each excimer lamp 13.

The excimer lamp 13 has a light-emitting tube with the Y-direction as the tube axis direction, and the outer surface of the light-emitting tube of the excimer lamp 13 is in contact with each electrode block (15, 16) at a separated position in the Y-direction. The light-emitting tube of the excimer lamp 13 is filled with the light-emitting gas G13. Based on the control from the control unit 3 (see FIG. 7), when a high frequency AC voltage of, for example, several kHz to 5 MHz is applied between the electrode blocks (15, 16) from the power supply unit 4 through the power feed wires 18 (see FIG. 8), the voltage is applied to the light-emitting gas G13 through the light-emitting tube of the excimer lamp 13. This applied voltage induces discharge plasma in the discharge space where the light-emitting gas G13 is enclosed, excites the atoms in the light-emitting gas G13 to the excimer state, and generates excimer emission when these atoms shift to the ground state.

The light-emitting gas G13 is composed of a material having a peak wavelength in the wavelength band of 200 nm or more and less than 240 nm, and a light intensity suppressed in the band of 250 nm or more and less than 400 nm during excimer light emission; and emits the ultraviolet light L10. Examples of the light-emitting gas G13 include KrCl and KrBr.

In the case in which the light-emitting gas G13 contains KrCl, for example, the excimer lamp 13 emits the ultraviolet light L10 having a peak wavelength near 222 nm. In the case in which the light-emitting gas G13 contains KrBr, for example, the excimer lamp 13 emits the ultraviolet light L10 having a peak wavelength near 207 nm. In the case that the light-emitting gas G13 contains these gases, the ultraviolet light L10 does not emit light that contains substantial light intensity in the band of 250 nm or more and less than 400 nm. It is noted that the term "near 222 nm" is intended to include individual differences in manufacturing excimer lamps, and to permit wavelength deviations within a range of ±3 nm with respect to 222 nm, as well as 222.0 nm.

FIG.11 is a graph illustrating the spectrum of the ultraviolet light L10 emitted from the first light source 10 when the first light source 10 is provided with an excimer lamp 13 in which the light-emitting gas G13 containing KrCl is filled in the light-emitting tube. As shown in FIG. 11, in the case of the excimer lamp 13 in which the light-emitting gas G13 containing KrCl is filled in the light-emitting tube, the light intensity in the band from 250 nm or more and less than 400 nm is suppressed. In other words, the light intensity in the wavelength band from 250 nm or more and less than 400 nm in the ultraviolet light L10 emitted from the first light source 10 is 1 mW/cm² or less, and is less than 5% with respect to the light intensity at the peak wavelength (in this case, near 222 nm), as described above with reference to FIG. 3.

It is more desirable to appropriately suppress the light intensity in a wavelength band of 240 nm or more and less than 300 nm since the light has a risk of adversely affecting the human body when being irradiated thereto. Hence, the first light source 10 may be provided with an optical filter, for example, that transmits ultraviolet light in the band of 200 nm or more and less than 240 nm (more preferably, ultraviolet light in the band of 200 nm or more and less than 235 nm) while blocking ultraviolet light in the band of 240 nm or more and less than 300 nm. Examples of the optical filter include a dielectric multilayer film made of HfO₂ layers and SiO₂ layers. The similar consideration can be applied to a case in which the excimer lamp 13 mounted on the first light source 10 has a light-emitting tube in which a light-emitting gas G13 other than KrCl, for example KrBr, is filled.

The ultraviolet light L10 emitted from the first light source 10 has a light intensity suppressed in the band of 250 nm or more and less than 400 nm, and a peak wavelength in the wavelength band of 200 nm or more and less than 240 nm, thus ensuring its ability to inactivate bacteria or viruses. Hereinafter, this subject will be explained with reference to the verification results.

A petri dish with a diameter of 35 mm was filled with 1 ml of Staphylococcus aureus with a concentration of approximately 10⁶/mL, and was irradiated from above the petri dish with the ultraviolet light L10 having the spectrum shown in Fig. 11 under different illumination conditions. The solution in the petri dish, which had been irradiated with the ultraviolet light L10, was diluted to a predetermined magnification with saline solution. The diluted solution of 0.1 mL was seeded onto a standard agar medium and cultured for 24 hours under the culture environment of 37°C temperature and 70% humidity, then the number of colonies was counted.

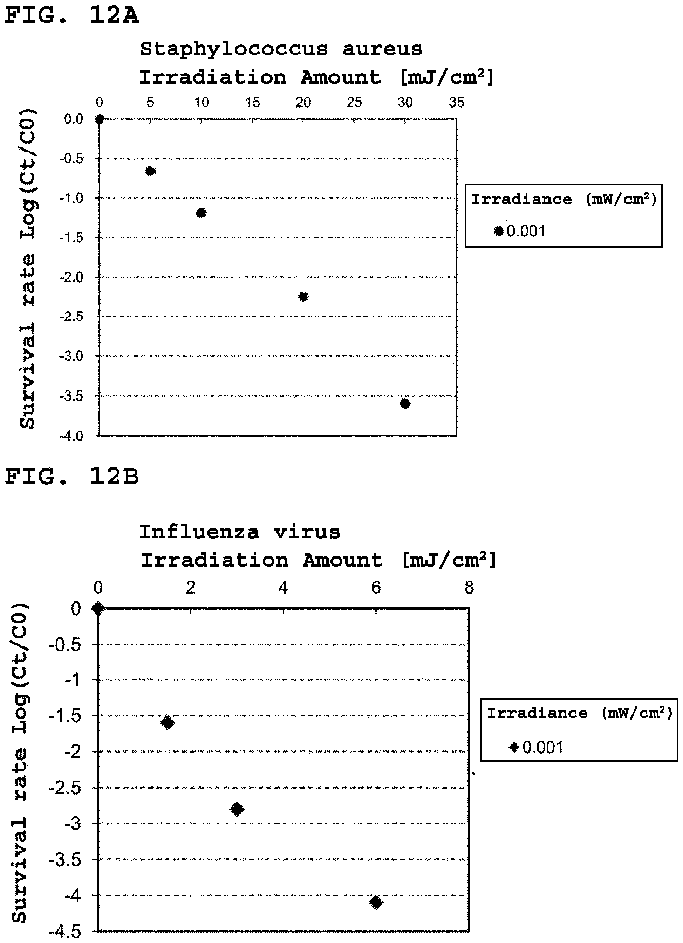

FIG. 12A is a graph of the result on the above experimental procedure, with the horizontal axis corresponding to the amount of ultraviolet light L10 irradiated and the vertical axis corresponding to the survival rate of Staphylococcus aureus. It is noted that the vertical axis represents the ratio of the number of colonies of Staphylococcus aureus after the irradiation of the ultraviolet light L10 to the number of colonies of Staphylococcus aureus before the irradiation, the ratio being in a log scale.

FIG. 12A confirms that Staphylococcus aureus is successfully inactivated even when the irradiance of the ultraviolet light L10 is extremely low, such as 0.001mW/cm². The ultraviolet light L10 has also been confirmed to have an effect of inactivation on other bacteria such as Bacillus cereus and Bacillus subtilis.

As another verification, FIG. 12B is a graph of the verification results for influenza virus using the similar experimental procedure. FIG. 12B confirms that influenza virus is successfully inactivated by the irradiation of the ultraviolet light L10. Achieving an irradiation amount of the ultraviolet light L10, for example, of 3 mJ/cm², requires irradiation time of 50 minutes in the case of an irradiance of 0.001 mW/cm², whereas 5 minutes in the case of an irradiance of 0.01 mW/cm². It is noted that the ultraviolet light L10 has also been confirmed to have an effect of inactivation on other viruses such as feline coronavirus. Therefore, the ultraviolet light L10 is confirmed to have an effect of inactivation on viruses as well as bacteria.

The extent of the inactivation effect of bacteria or viruses depends on the accumulated irradiation amount (dose) of the irradiated ultraviolet light L10.

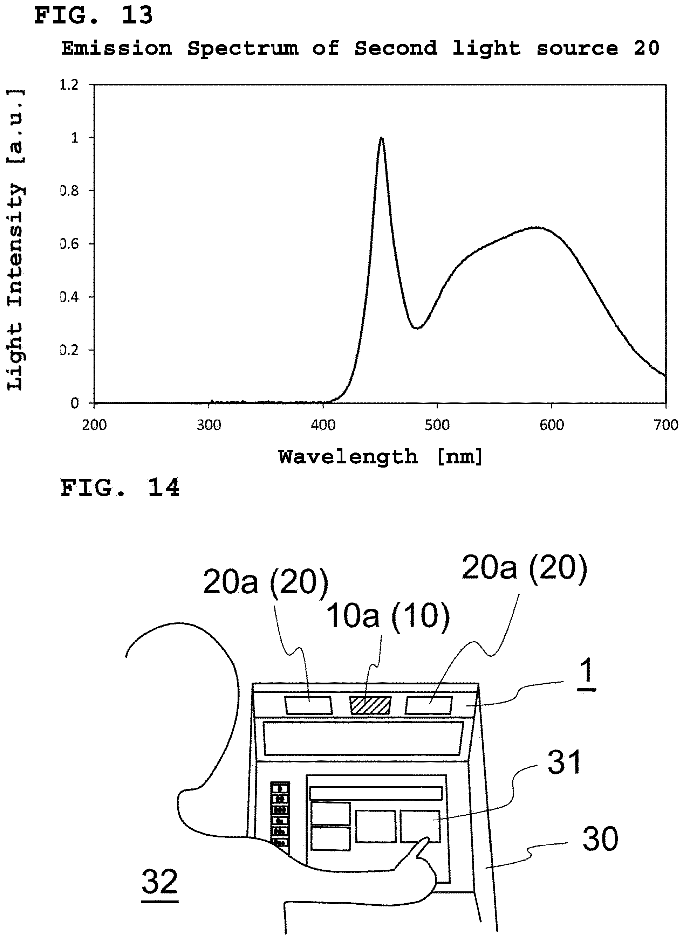

The second light source 20 includes an LED element that emits white light L20 for illumination. FIG. 13 is a graph illustrating an example of the spectrum of white light L20 emitted from the LED element. In this example, the second light source 20 includes a blue LED element with a peak wavelength near 450 nm, and a phosphor that is excited by the blue light emitted from the blue LED element and emits fluorescence with a longer wavelength including yellow band. In the example shown in FIG. 13, the white light L20 has a light intensity suppressed in a wavelength band of 300 nm or more and less than 400 nm.

In other words, the lighting device with inactivation function 1 is capable of inactivating bacteria or viruses that are present in the irradiation target area 40 by irradiating the irradiation target area 40 with ultraviolet light L10 for inactivation. This ultraviolet light L10 has a light intensity suppressed in the wavelength band of 300 nm or more and less than 400 nm, in which insects are known to have relatively high visual sensitivity. Consequently, even when the first light source 10 is turned on for inactivation treatment, the ultraviolet light L10 emitted from this first light source 10 is inhibited from attracting insects.

Furthermore, this ultraviolet light L10 has light intensity suppressed in a wavelength band of 240 nm or more and less than 300 nm. Hence, the lighting device with inactivation function 1 is capable of performing the inactivation process even during the time when humans are present near the irradiation target area 40.

Furthermore, this lighting device with inactivation function 1 irradiates the irradiation target area 40 with the white light L20 emitted from the LED element that constitutes the second light source 20. As shown in FIG. 13, this white light L20 has light suppressed in the wavelength band of 300 nm or more and less than 400 nm compared to the white light emitted from a fluorescent lamp described above with reference to FIG. 4. Consequently, even when the second light source 20 is turned on for illumination, the white light L20 emitted from the second light source 20 is inhibited from attracting insects.

In other words, the lighting system with inactivation function 1 is capable of inactivating bacteria or viruses while inhibiting the effect of attracting insects, especially in the case that the area to be irradiated overlaps with the area to be inactivated, or in other words, in the case that the irradiation target area 40 is irradiated with both the ultraviolet light L10 and the white light L20, as described above with reference to FIG. 5.

The lighting device with the inactivation function 1 in the shape shown in FIG. 6 can be installed, for example, on an indoor ceiling or an indoor wall for lighting while inactivating bacteria or viruses present in indoor spaces and on fixtures such as desks and chairs.

As another example shown in FIG. 14, the lighting device with the inactivation function 1 may find applications in irradiating objects operated by person's fingers. FIG. 14 schematically illustrates the state in which the lighting device with the inactivation function 1 is mounted on a ticket vending machine 30 as an example of the object.

A second light source 20, which is provided in a lighting system with inactivation function 1, irradiates white light L20 from a light extraction surface 20a to a touch panel 31, so that the white light is used for illumination when an operator 32 operates the touch panel 31. Moreover, since the touch panel 31 is intended to be operated by a plurality of operators 32, it is likely to have bacteria or viruses attached thereto. In contrast, a first light source 10, which is provided in the lighting system with the inactivation function 1, irradiates ultraviolet light L10 from a light extraction surface 10a to the touch panel 31, thereby inactivating bacteria or viruses attached to the touch panel 31.

As described above, since the ultraviolet light L10 does not exhibit substantial light intensity in the wavelength band of 240 nm or more and less than 300 nm, it inhibits the adverse effect on the human body of the operator 32 even if the ultraviolet light L10 is irradiated during the time when the operator 32 is present near the touch panel 31.

In addition, even when the lighting device with the inactivation function 1 operates under dark surroundings such as at night and irradiates the touch panel 31 with the ultraviolet light L10 for inactivation and the white light L20 for illumination, insects are unlikely to be attracted near the ticket vending machine 30 including the touch panel 31.

It is preferable that the first light source 10 operate intermittently from the viewpoint of further enhancing the effect of inhibiting insects from being attracted. Specifically, the control unit 3 may control the first light source 10 to turn on and off repetitively. In the case that the ultraviolet light L10 emitted from the first light source 10 contains a weak amount of light in the wavelength band of 250 nm or more and less than 400 nm, the weak light relatively stands out under dark surroundings, thus may lead to attracting insects that possess extremely high phototaxis in response to this light. However, when the first light source 10 is temporarily turned off, insects that have once been attracted to the lighting device with the inactivation function 1 lose sight of the location (target) of the light source and tend to proceed toward other locations. Therefore, the effect of attracting insects is further reduced.

From the viewpoint of enhancing the effect of causing insects to lose sight of the target, it is preferable to set the lighting-on period of the first light source 10 to be 60 seconds or less, and then to set the lighting-off period to be longer than the lighting-on period. The control contents may be stored in the control unit 3 in advance.

Intermittent lighting control on the first light source 10 is effective when the second light source 20 is turned off, especially when the white light L20 for illumination is not irradiated. The reason for this effectiveness is that the surroundings become a brighter environment when the second light source 20 is turned on, making the relatively weak light less noticeable. In other words, the control unit 3 may perform intermittent lighting control to the first light source 10 when the control unit 3 detects the second light source 20 being turned off.

As described above, the extent of the inactivation effect of bacteria or viruses depends on the accumulated irradiation amount (dose) of the irradiated ultraviolet light L10. Hence, even when the first light source 10 operates intermittently, bacteria or viruses that are present in the irradiation target area 40 is effectively inactivated provided that the accumulated irradiation amount of ultraviolet light L10 from the first light source 10 is secured to be irradiated to the irradiation target area 40. In other words, the intermittent lighting of the first light source 10 does not mean that bacteria or viruses cannot be effectively inactivated.

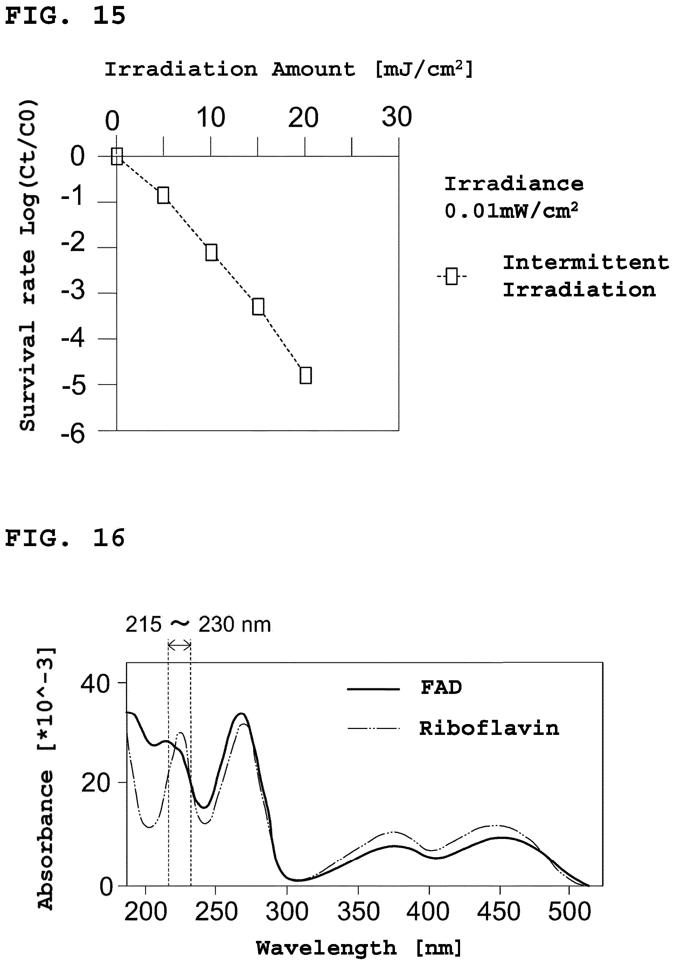

FIG. 15 is a graph illustrating the verification result of inactivating Staphylococcus aureus using the similar procedure as in FIG. 12A, except the ultraviolet light L10 operating intermittently. The irradiation conditions employed were intermittent lighting at a duty ratio of 50% (lighting-on for 8.3 minutes/ lighting-off for 8.3 minutes) with an illuminance during lighting-on of 0.01 mW/cm².

The result in FIG. 15 indicates that inactivation of germs can be achieved even in the case of the intermittent irradiation of ultraviolet light L10.

Furthermore, the ultraviolet light in the wavelength band of 200 nm or more and less than 240 nm, in which the insect inducing properties are effectively reduced, and especially in the wavelength band of 200 nm or more and less than 235 nm, and more preferably in the wavelength band of 200 nm or more and less than 230 nm, is absorbed by the stratum corneum of the skin and does not penetrate to the further inner layer (basal layer side), even when irradiated to the human body, causing virtually no adverse effect on the human body. This makes it effective in inactivating bacteria or viruses in spaces where people come and go or on surfaces of objects.

In the cells of germs, there are nucleic acids (DNA, RNA) that contain genetic information. When germs are irradiated with ultraviolet light, the nucleic acids contained therein absorb the ultraviolet light, damaging the binding of DNA and RNA. The damage interferes with the transcriptional control by the gene, which hinders metabolism and leads to death. In other words, when germs are irradiated with ultraviolet light, the DNA and RNA contained in the germs are damaged by the ultraviolet light, resulting in losing the ability of metabolism and proliferation, and thereby killing the germs.

However, when germs have been inactivated by irradiating with ultraviolet light, for example, with a wavelength of 254 nm, and are irradiated with light in a wavelength band of 300 nm or more and 500 nm or less, some germs exhibit the repair of their damaged DNA. This phenomenon is caused by the activity of photoreactivation enzymes (e.g., FAD (flavin adenine dinucleotide)) that bacteria possess, and is referred to as "photoreactivation of bacteria" below. The wavelength band of 300 nm or more and 500 nm or less includes that of the sunlight and the visible light of white lighting, and it is known that the photoreactivation of bacteria proceeds under a bright environment. In the case of inactivating germs by irradiating them with ultraviolet light under the lighting environment, maintaining the inactivation state tends to be difficult because of the photoreactivation.

In contrast, in the case of inactivating germs by irradiating them with ultraviolet light in the wavelength band of 200 nm or more and 235 nm or less (especially ultraviolet light with a peak wavelength near 222 nm), it is confirmed that no "bacteria photoreactivation" is performed even when the above visible light is irradiated after the ultraviolet light has been irradiated; in other words, "bacteria photoreactivation" is confirmed to be inhibited.

FAD, which is a photoreactivating enzyme, is classified into riboflavin, which acts on photoreactivation, and ADP (adenine nucleotide). ADP is further classified into adenosine and phosphate. FIG. 16 is a graph illustrating the absorption spectra of FAD and riboflavin. FIG. 16 indicates that the absorbance of FAD with respect to ultraviolet light of wavelength 222 nm is almost equal to that with respect to ultraviolet light of wavelength 254 nm. In contrast, the absorbance of riboflavin, which acts on photoreactivation, with respect to ultraviolet light of wavelength band of 215 nm or more and 230 nm or less is higher than that with respect to ultraviolet light of wavelength 254 nm.

In other words, it is inferred that when ultraviolet light in the wavelength band between 215 nm and 230 nm is irradiated to germs, the ultraviolet light effectively acts on the riboflavin contained in the FADs that germs possess, resulting in inhibiting bacteria photoreactivation from functioning. Furthermore, FIG. 16 indicates that the peak absorbance value of riboflavin is located near 222 nm, leading to an inference that the irradiation of ultraviolet light with a peak wavelength near 222 nm greatly inhibits the "bacteria photoreactivation".

With regard to adenosine, the absorbance with respect to ultraviolet light in the wavelength of 254 nm is higher than that with respect to ultraviolet light in a wavelength band between 218 nm and 245 nm. In other words, ultraviolet light with a wavelength of 254 nm is readily absorbed by adenosine, so that it is inferred that adenosine acts as a protective barrier that prevents riboflavin from acting effectively. On the contrary, it is inferred that ultraviolet light in the wavelength band of 218 nm or more and 245 nm or less is more likely to effectively act on riboflavin. From the above discussion, ultraviolet light near the wavelength of 222 nm is light that satisfies both of the above effective wavelength ranges, and is considered to effectively inhibit the photoreactivation effect of germs.

Hereinafter, the irradiation of ultraviolet light having different wavelengths that influence the photoreactivation effect of germs will be explained with reference to experimental results.

### (Method of experiment)

Staphylococcus aureus, which is a target for inactivation, was irradiated with ultraviolet light for inactivation for a certain period of time (in this case, 30 minutes) under an environment in which visible light containing a wavelength band between 300 nm and 500 nm was irradiated (under lighting environment), and afterward the ultraviolet light irradiation stopped. Then, the visible light irradiation continued for the period described below, and Staphylococcus aureus was cultured to confirm the variation in the survival rate thereof. The ultraviolet light used for inactivation was ultraviolet light from a low-pressure mercury lamp with a peak wavelength near 254 nm (Comparative Example 1) as shown in FIG. 1, and ultraviolet light from a KrCl excimer lamp with a peak wavelength near 222 nm (Example 1) as shown in FIG. 11.

### (Result Analysis)

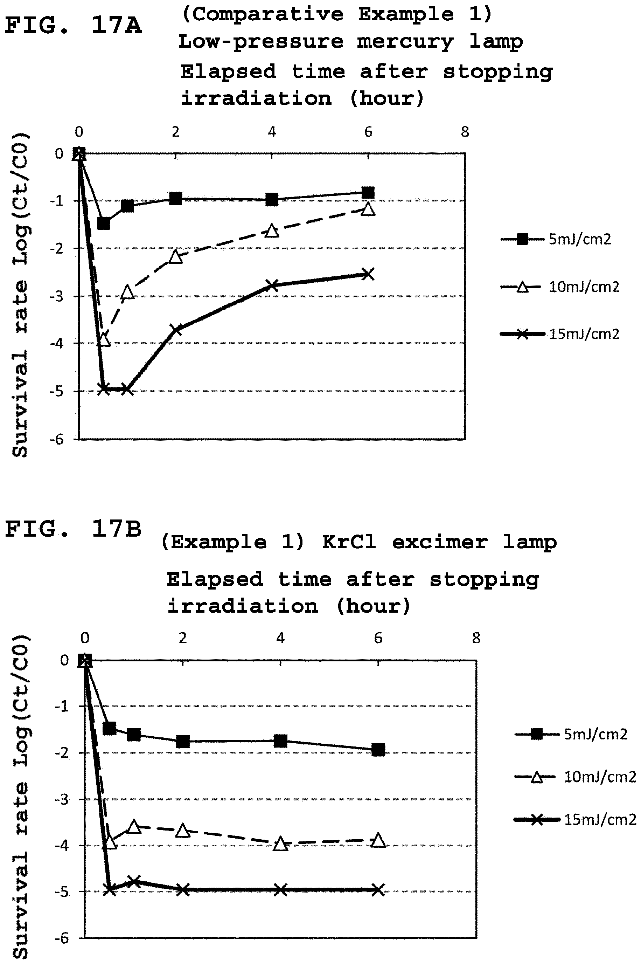

FIG. 17A is a graph indicating the variation in the survival rate of bacteria in the case of Comparative Example 1. FIG. 17B is a graph indicating the variation in the survival rate of bacteria in the case of Example 1. In both cases, the variation in the survival rate of bacteria is shown when the illuminance of ultraviolet light is set to 5 mJ/cm², 10 mJ/cm², and 15 mJ/cm². In other words, for both Comparative Example 1 and Example 1, the experiments were conducted with ultraviolet light being irradiated for the same period of time at three different illuminances, and with different elapsed times (irradiation time of visible light) after stopping the ultraviolet light irradiation. In FIG. 17A and 17B, the vertical axis corresponds to the ratio of the number of colonies of Staphylococcus aureus after irradiation (Ct) to the number of colonies of Staphylococcus aureus at the time before irradiation (C0), the ratio being in a log scale.

The result in FIG. 17A indicates that the survival rate of the bacteria exhibits an upward trend with the elapse of time after stopping the ultraviolet light irradiation in a case of the low-pressure mercury lamp being used to inactivate the bacteria. This result suggests that, under an environment of visible light irradiation, the bacteria are being photoreactivated during stopping the irradiation of ultraviolet light with a peak wavelength near 254 nm after the ultraviolet light irradiation. Specifically, the number of surviving bacteria rises with the irradiation duration of the visible light after stopping the ultraviolet light irradiation, and recovers dramatically after elapse of approximately one to two hours after stopping the ultraviolet light irradiation.

In contrast, the result in FIG. 17B indicates that the survival rate of the bacteria remains substantially constant with the elapse of time after stopping the ultraviolet light irradiation in a case of the KrCl excimer lamp being used to inactivate the bacteria. This result suggests that, even under an environment of visible light irradiation, irradiating the bacteria with ultraviolet light near the peak wavelength of 222 nm inhibits their photoreactivation.

Bacteria with impaired photoreactivation will be dead (inactivated) without repairing its DNA since the DNA damage remains. Hence, irradiating bacteria with ultraviolet light near the wavelength of 222 nm effectively inhibits the recovery and the growth of bacteria. Therefore, devices and systems that use ultraviolet light near a wavelength of 222 nm as ultraviolet light for inactivation are particularly effective in an environment in which bacteria are likely to be photoreactivated, specifically in an environment in which visible light, including light with a wavelength between 300 nm and 500 nm, is irradiated.

Devices and systems that inactivate germs by irradiating them with ultraviolet light of a wavelength of 254 nm effectively inactivate bacteria or viruses that do not recover from light (e.g., Bacillus subtilis (so-called natto bacteria), influenza, etc.); however, they have difficulty in continuously inactivating bacteria that recover from light (e.g., Escherichia coli, Salmonella, etc.) in an environment of visible light irradiation. Therefore, using these devices and systems for inactivating germs is likely to create an environment in which specific bacteria with photoreactivation enzymes can survive, leading to concern for increasing the infection risk of such bacteria.

However, as described above, irradiation with ultraviolet light in the wavelength band between 200 nm and 230 nm, especially ultraviolet light near the wavelength of 222 nm, is capable of inhibiting the photoreactivation function of harmful bacteria that have photoreactivation enzymes, thus reducing the infection risk of bacteria.

Also, inhibiting the photoreactivation of the bacteria will inhibit the growth of viruses mediated by the bacteria. For example, viruses (bacteriophages) that infect bacteria are known to grow in bacteria as a vector. This bacteriophage is a general term for viruses that infect bacteria, but can also be harmful to humans. For example, lysogenic phages occasionally have toxic or drug resistance genes in their genomes, and these genes have been noted to have a potential to harm humans indirectly through bacteria. Examples are the toxins of cholera and diphtheria. Inhibiting the bacteria photoreactivation also leads to preventing the growth of viruses such as phages.

As described above, the lighting device with the inactivation function 1 provided with the first light source 10 that emits ultraviolet light L10 in the wavelength band of 200 nm or more and less than 240 nm, and especially in the wavelength band of 200 nm or more and 230 nm or less, inactivates harmful bacteria or viruses present in a space or on the surface of an object, and effectively inhibits the bacteria photoreactivation after ultraviolet light irradiation, even when white light L20 for illumination is irradiated from the second light source 20. This is an advantageous effect as an inactivation function that is added to the lighting system.

In addition, since the irradiation of ultraviolet light with a wavelength band between 200 nm and 230 nm, and especially ultraviolet light with a wavelength near 222 nm, inhibits the bacteria photoreactivation, the effect of inactivation is easily maintained even in the case in which a time period (pause time) of stopping the irradiation of the ultraviolet light L10 from the first light source 10 is provided, while white light L20 for illumination being irradiated from the second light source 20.

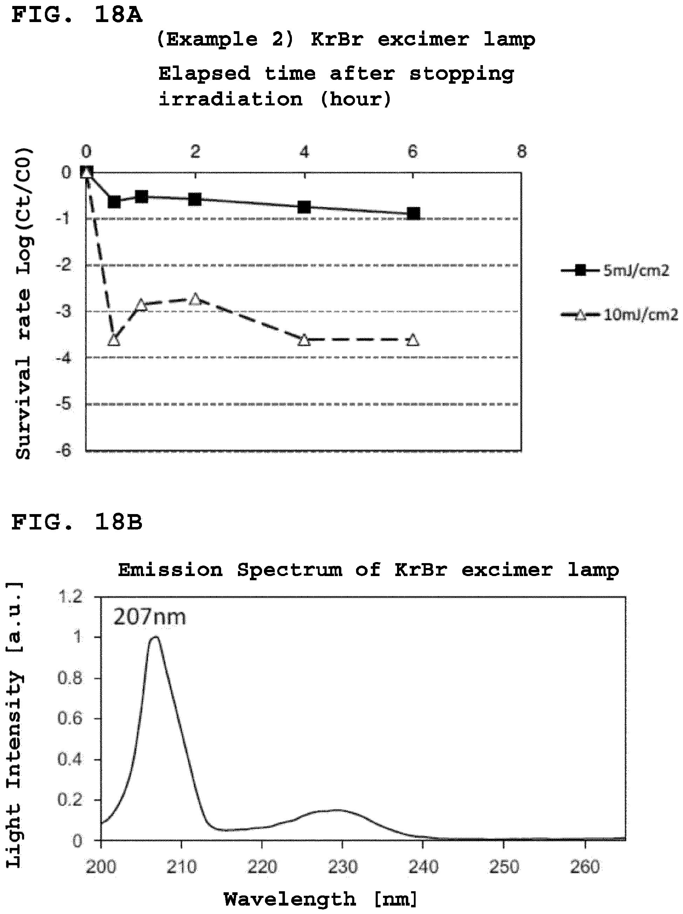

Furthermore, the inventors' intensive research confirms that the irradiation of ultraviolet light with a peak wavelength of 207 nm also inhibits the photoreactivation function, unlike the irradiation of ultraviolet light with a wavelength of 254 nm. Figure 18A is a graph illustrating variation in the survival rate of bacteria when ultraviolet light from a KrBr excimer lamp with a peak wavelength of approximately 207 nm (Example 2) is used as ultraviolet light for inactivation. Note that the method of measuring data and the method of showing graphs are the same as those used in Example 1 and Comparative Example 1. FIG. 18B is a graph illustrating the emission spectrum of a KrBr excimer lamp used in Example 2.

The result in FIG. 18A indicates that the irradiation of ultraviolet light with a peak wavelength of 207 nm also exhibits the effect of inhibiting the bacteria photoreactivation, which is similar to the irradiation result of ultraviolet light with a peak wavelength of 222 nm shown in FIG. 17B. These results suggest that ultraviolet light with a wavelength of less than 240 nm has an apparent effect on the cellular tissues that constitute bacteria or viruses.

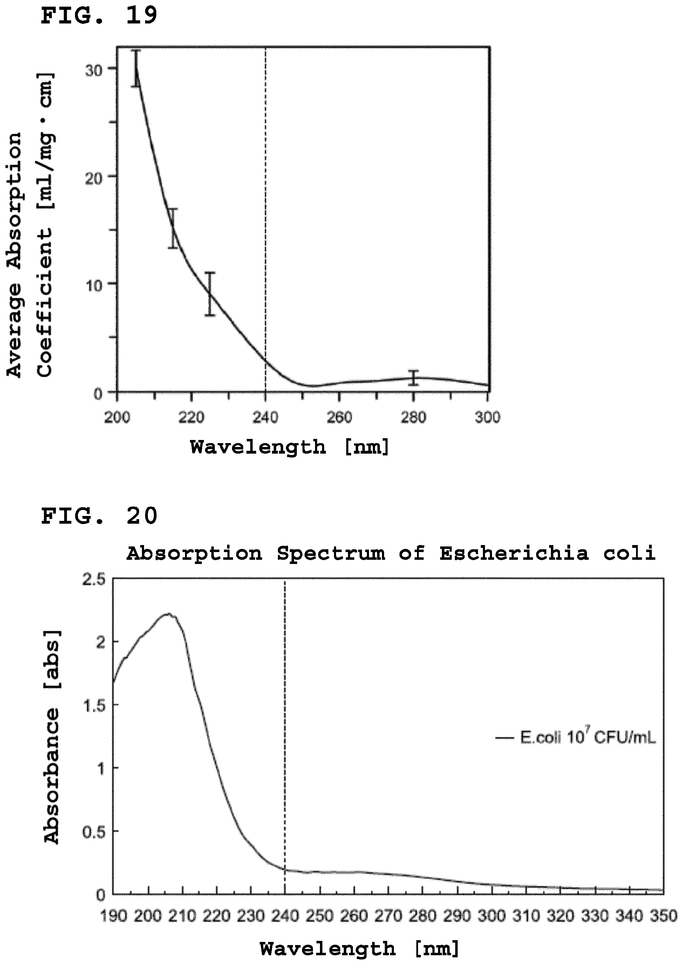

FIG. 19 is a graph illustrating the characteristics of the average absorption coefficient of protein in the wavelength band of ultraviolet light. As shown in FIG. 19, protein does not readily absorb ultraviolet light at wavelengths above 250 nm, but in the band of wavelengths below 240 nm, the tendency of protein to absorb ultraviolet light increases sharply at shorter wavelengths. Hence, ultraviolet light with a wavelength of less than 240 nm, such as ultraviolet light from KrCl excimer lamps or KrBr excimer lamps, is effectively absorbed by protein, which are components of the cell membranes and enzymes of bacteria or viruses. Ultraviolet light in this band is absorbed at the surface of human skin (e.g., stratum corneum) and is difficult to penetrate into the skin, thereby ensuring safety for the skin. In contrast, since bacteria or viruses are physically much smaller than human cells, ultraviolet light can easily reach the inside of them even in a wavelength band shorter than 240 nm. Therefore, ultraviolet light with a wavelength of less than 240 nm is considered to act effectively on cells that constitute bacteria or viruses, especially including cell membranes or enzymes that contain protein components, thus enhancing the effect of inhibiting functions such as bacteria photoreactivation.

Furthermore, ultraviolet light in a wavelength band between 215 nm and 230 nm has an apparent effect on riboflavin contained in the FAD that germs possess, leading to infer that complex reasons inhibit the photoreactivation of the bacteria.

Here, the absorbance of the prepared stock solution of Escherichia coli was measured in order to confirm the absorbance characteristics on protein. The measurement method of absorbance and the preparation method of stock solution of Escherichia coli are as follows.

Escherichia coli (NBRC. 106373 lyophilization product) was suspended in an LB medium and cultured at 37°C for 24 hours with shaking. Next, the above suspension was diluted to a range of 1/10⁵ to 1/10⁷ in the LB medium. The diluted suspension of 0.1mL was smeared onto a standard agar medium, and cultured at 37°C for 24 hours. In addition, one colony was fished from the standard agar medium of 30 to 300 CFU/Plate with a platinum ear, suspended in an LB medium of 5mL, and cultured at 37°C for 24 hours with shaking. The suspension was centrifugally cleaned with sterile physiological saline to serve as stock solution of Escherichia coli. The concentration of the stock solution obtained in the above procedures is 10⁹ CFU/mL. Absorbance measurement was conducted with NanoDrop of Thermo Fisher Scientific Inc., using a reagent of concentration of 10⁷ CFU/mL, which is diluted to 1/100 of the stock solution.

FIG. 20 is a graph illustrating the absorption spectrum of Escherichia coli (E. coli). FIG. 20 indicates that the absorbance of Escherichia coli (E. coli) increases for light in the wavelength band of shorter than 240 nm, similar to the tendency of the average absorbance coefficient of protein. This result suggests that ultraviolet light with a wavelength band shorter than 240 nm has a more apparent effect on the cellular tissues that constitute bacteria, viruses or the like.

Compared with conventional inactivation devices using ultraviolet light, ultraviolet light in the wavelength band of less than 240 nm provides a favorable inactivation effect even with intermittent irradiation of ultraviolet light by inhibiting the bacteria photoreactivation. In other words, even in the case of performing inactivation with moving the irradiation position of the ultraviolet light, the inactivation still proceeds efficiently. From the viewpoint of more effectively inhibiting the bacteria photoreactivation, it is more preferable that the peak wavelength of the ultraviolet light emitted from the light source section be in a wavelength band between 200 nm and 235 nm.

### [Another embodiment] Hereinafter, another embodiment is described.

<1 > The lighting system with inactivation function 1 may be configured to merely control to turn on and off each of the first light source 10 and the second light source 20.
<2> In the above embodiments, the case in which the first light source 10 includes the excimer lamp 13 is explained. However, the configuration of the first light source 10 can be anything as long as the resultant ultraviolet light L10 has a peak wavelength in a wavelength band of 200 nm or more and less than 240 nm, and has a light intensity suppressed in a band of 250 nm or more and less than 400 nm. The ultraviolet light L10 emitted from the first light source 10 is preferably light having a narrow band spectrum, thus suitably using light sources including excimer lamps, LED elements and LD elements. Further, in the case of using a light source that emits the ultraviolet light L10 with a broad emission spectral shape as the first light source 10, a filter or the like may be provided to block the light with the wavelength band to be suppressed.

### REFERENCE SIGN LIST

- 1: lighting device for inactivating bacteria or viruses
- 2: casing
- 3: control unit
- 4: power supply unit
- 10: first light source
- 10a: light extraction surface of first light source
- 12: lamp house
- 12a: main body casing part
- 12b: lid part
- 13: excimer lamp
- 18: feed wires
- 20: second light source
- 20a: light extraction surface of first light source
- 30: ticket vending machine
- 31: touch panel
- 32: operator
- 40: irradiation target area
- G13: light-emitting gas
- L10: ultraviolet light
- L20: white light

## Claims

1. A lighting device for inactivating bacteria or viruses (1), comprising:
a first light source (10) for emitting ultraviolet light (L10) with a peak wavelength in a wavelength band of 200 nm or more and less than 240 nm and with light intensity suppressed in a wavelength band of 250 nm or more and less than 400 nm; and
a second light source (20) that is composed of an LED element, for emitting white light (L20) for illumination.

2. The lighting device for inactivating bacteria or viruses (1) according to claim 1, wherein the first light source (10) irradiates an area with the ultraviolet light (L10), the area being irradiated by the second light source (20).

3. The lighting device for inactivating bacteria or viruses (1) according to claim 2, wherein the second light source (20) irradiates an object operated by a person with the white light (L20); and the first light source (10) irradiates the object with the ultraviolet light (L10) to inactivate bacteria or viruses attached to the object.

4. The lighting device for inactivating bacteria or viruses (1) according to any one of claims 1 to 3, wherein the ultraviolet light (L10) from the first light source (10) has light intensity suppressed in a wavelength band of 240 nm or more and less than 400 nm.

5. The lighting device for inactivating bacteria or viruses (1) according to any one of claims 1 to 4, further comprising a control unit (3) for controlling to light the first light source (10) and the second light source (20), wherein the control unit (3) controls to repeatedly turn on and off the first light source (10) within a lighting-off period of the second light source (20).

6. The lighting device for inactivating bacteria or viruses (1) according to claim 5, wherein the control unit (3) controls to repeatedly turn on and off the first light source (10), with a lighting-on period of the first light source (10) being set to 60 seconds or less, and a lighting-off period of the first light source (10) being set to a time longer than the lighting-on period thereof.

7. The lighting device for inactivating bacteria or viruses (1) according to claim 6, wherein the control unit (3) controls to repeatedly turn on and off the first light source (10) with the lighting-on period of the first light source (10) being set to 50% or less with respect to the lighting-off period of the first light source (10).

8. The lighting device for inactivating bacteria or viruses (1) according to any one of claims 1 to 4, further comprising a control unit (3) for controlling to light the first light source (10) and the second light source (20), wherein the control unit (3) controls the first light source (10) to provide the lighting-off period of the first light source (10) within a lighting-on period of the second light source (20).

9. The lighting device for inactivating bacteria or viruses (1) according to claim 8, wherein the control unit (3) controls repeatedly to turn on and off the first light source (10).

10. The lighting device for inactivating bacteria or viruses (1) according to any one of claims 1 to 9, wherein the first light source (10) includes an excimer lamp (13) that contains Kr and Cl as light-emitting gases.

11. The lighting device for inactivating bacteria or viruses (1) according to any one of claims 1 to 9, wherein the first light source (10) includes an excimer lamp (13) that contains Kr and Br as light-emitting gases.

## Patentansprüche

1. Beleuchtungsvorrichtung zum Inaktivieren von Bakterien oder Viren (1), umfassend:
eine erste Lichtquelle (10) zum Emittieren von ultraviolettem Licht (L10) mit einer Peak-Wellenlänge in einem Wellenlängenband von 200 nm oder mehr und weniger als 240 nm und mit einer Lichtintensität, die in einem Wellenlängenband von 250 nm oder mehr und weniger als 400 nm unterdrückt ist, und
eine zweite Lichtquelle (20), die aus einem LED-Element besteht, um weißes Licht (L20) zur Beleuchtung zu emittieren.

2. Beleuchtungsvorrichtung zum Inaktivieren von Bakterien oder Viren (1) nach Anspruch 1, wobei die erste Lichtquelle (10) einen Bereich mit dem ultravioletten Licht (L10) bestrahlt, wobei der Bereich von der zweiten Lichtquelle (20) bestrahlt wird.

3. Beleuchtungsvorrichtung zum Inaktivieren von Bakterien oder Viren (1) nach Anspruch 2, wobei die zweite Lichtquelle (20) ein von einer Person bedientes Objekt mit dem weißen Licht (L20) bestrahlt und die erste Lichtquelle (10) das Objekt mit dem ultravioletten Licht (L10) bestrahlt, um an dem Objekt haftende Bakterien oder Viren zu inaktivieren.

4. Beleuchtungsvorrichtung zum Inaktivieren von Bakterien oder Viren (1) nach einem der Ansprüche 1 bis 3, wobei das ultraviolette Licht (L10) von der ersten Lichtquelle (10) eine Lichtintensität aufweist, die in einem Wellenlängenband von 240 nm oder mehr und weniger als 400 nm unterdrückt ist.

5. Beleuchtungsvorrichtung zum Inaktivieren von Bakterien oder Viren (1) nach einem der Ansprüche 1 bis 4, weiter umfassend eine Steuereinheit (3) zum Steuern des Einschaltens der ersten Lichtquelle (10) und der zweiten Lichtquelle (20), wobei die Steuereinheit (3) das wiederholte Einund Ausschalten der ersten Lichtquelle (10) innerhalb eines Ausschaltzeitraums der zweiten Lichtquelle (20) steuert.

6. Beleuchtungsvorrichtung zum Inaktivieren von Bakterien oder Viren (1) nach Anspruch 5, wobei die Steuereinheit (3) das wiederholte Ein- und Ausschalten der ersten Lichtquelle (10) steuert, wobei ein Einschaltzeitraum der ersten Lichtquelle (10) auf 60 Sekunden oder weniger und ein Ausschaltzeitraum der ersten Lichtquelle (10) auf eine Zeit eingestellt ist, die länger ist als deren Einschaltzeitraum.

7. Beleuchtungsvorrichtung zum Inaktivieren von Bakterien oder Viren (1) nach Anspruch 6, wobei die Steuereinheit (3) das wiederholte Ein- und Ausschalten der ersten Lichtquelle (10) steuert, wobei der Einschaltzeitraum der ersten Lichtquelle (10) auf 50 % oder weniger in Bezug auf den Ausschaltzeitraum der ersten Lichtquelle (10) eingestellt ist.

8. Beleuchtungsvorrichtung zum Inaktivieren von Bakterien oder Viren (1) nach einem der Ansprüche 1 bis 4, weiter umfassend eine Steuereinheit (3) zum Steuern des Einschaltens der ersten Lichtquelle (10) und der zweiten Lichtquelle (20), wobei die Steuereinheit (3) die erste Lichtquelle (10) so steuert, dass der Ausschaltzeitraum der ersten Lichtquelle (10) innerhalb eines Einschaltzeitraums der zweiten Lichtquelle (20) liegt.

9. Beleuchtungsvorrichtung zum Inaktivieren von Bakterien oder Viren (1) nach Anspruch 8, wobei die Steuereinheit (3) das wiederholte Ein- und Ausschalten der ersten Lichtquelle (10) steuert.

10. Beleuchtungsvorrichtung zum Inaktivieren von Bakterien oder Viren (1) nach einem der Ansprüche 1 bis 9, wobei die erste Lichtquelle (10) eine Excimer-Lampe (13) umfasst, die Kr und Cl als lichtemittierende Gase enthält.

11. Beleuchtungsvorrichtung zum Inaktivieren von Bakterien oder Viren (1) nach einem der Ansprüche 1 bis 9, wobei die erste Lichtquelle (10) eine Excimer-Lampe (13) umfasst, die Kr und Br als lichtemittierende Gase enthält.

## Revendications

1. Dispositif d'éclairage destiné à inactiver des bactéries ou des virus (1), comprenant :
une première source de lumière (10) destinée à émettre une lumière ultraviolette (L10) avec une longueur d'onde de crête dans une bande de longueur d'onde supérieure ou égale à 200 nm et inférieure à 240 nm et avec une intensité lumineuse supprimée dans une bande de longueur d'onde supérieure ou égale à 250 nm et inférieure à 400 nm ; et
une seconde source de lumière (20) qui est composée d'un élément de LED destiné à émettre une lumière blanche (L20) pour l'illumination.

2. Dispositif d'éclairage d'inactivation de bactéries ou de virus (1) selon la revendication 1, dans lequel la première source de lumière (10) irradie une zone avec la lumière ultraviolette (L10), la zone étant irradiée par la seconde source de lumière (20).

3. Dispositif d'éclairage d'inactivation de bactéries ou de virus (1) selon la revendication 2, dans lequel la seconde source de lumière (20) irradie un objet manipulé par une personne avec la lumière blanche (L20) ; et la première source de lumière (10) irradie l'objet avec la lumière ultraviolette (L10) afin d'inactiver des bactéries ou des virus fixés à l'objet.

4. Dispositif d'éclairage d'inactivation de bactéries ou de virus (1) selon une quelconque des revendications 1 à 3, dans lequel la lumière ultraviolette (L10) en provenance de la première source de lumière (10) a une intensité lumineuse supprimée dans une bande de longueur d'onde supérieure ou égale à 240 nm et inférieure à 400 nm.

5. Dispositif d'éclairage d'inactivation de bactéries ou de virus (1) selon une quelconque des revendications 1 à 4, comprenant en outre une unité de commande (3) destinée à commander l'éclairage de la première source de lumière (10) et de la seconde source de lumière (20), dans lequel l'unité de commande (3) commande pour allumer et éteindre de manière répétée la première source de lumière (10) au cours d'une période d'extinction de la seconde source de lumière (20) .

6. Dispositif d'éclairage d'inactivation de bactéries ou de virus (1) selon la revendication 5, dans lequel l'unité de commande (3) commande pour allumer et éteindre de manière répétée la première source de lumière (10), avec une période d'allumage de la première source de lumière (10) qui est définie comme étant inférieure ou égale à 60 secondes, et une période d'extinction de la première source de lumière (10) qui est définie sur une durée plus longue que la période d'allumage de celle-ci.

7. Dispositif d'éclairage d'inactivation de bactéries ou de virus (1) selon la revendication 6, dans lequel l'unité de commande (3) commande pour allumer et éteindre de manière répétée la première source de lumière (10) avec la période d'allumage de la première source de lumière (10) qui est définie comme étant inférieure ou égale à 50 % par rapport à la période d'extinction de la première source de lumière (10) .

8. Dispositif d'éclairage d'inactivation de bactéries ou de virus (1) selon une quelconque des revendications 1 à 4, comprenant en outre une unité de commande (3) destinée à commander l'éclairage de la première source de lumière (10) et de la seconde source de lumière (20), dans lequel l'unité de commande (3) commande la première source de lumière (10) afin de fournir la période d'extinction de la première source de lumière (10) au cours d'une période d'allumage de la seconde source de lumière (20).

9. Dispositif d'éclairage d'inactivation de bactéries ou de virus (1) selon la revendication 8, dans lequel l'unité de commande (3) commande de manière répétée pour allumer et éteindre la première source de lumière (10).

10. Dispositif d'éclairage d'inactivation de bactéries ou de virus (1) selon une quelconque des revendications 1 à 9, dans lequel la première source de lumière (10) comporte une lampe à excimères (13) qui contient Kr et Cl comme gaz électroluminescents.

11. Dispositif d'éclairage d'inactivation de bactéries ou de virus (1) selon une quelconque des revendications 1 à 9, dans lequel la première source de lumière (10) comporte une lampe à excimères (13) qui contient Kr et Br comme gaz électroluminescents.
